# EUROPÄISCHE PATENTSCHRIFT

(11) **EP 1 418 908 B1**
(45) Veröffentlichungstag und Bekanntmachung des Hinweises auf die Patenterteilung: **26.09.2007**
(21) Anmeldenummer: 02758431.7
(22) Anmeldetag: 03.08.2002
(51) Int. Cl.: A61K 31/425, A61P 43/00

(54) **VERBINDUNGEN ZUR BESEITIGUNG UND/ODER LINDERUNG DER ANHEDONIE**
COMPOUNDS FOR ELIMINATING AND/OR RELIEVING ANHEDONIA
COMPOSES PERMETTANT DE SUPPRIMER ET/OU DE SOULAGER L'ANHEDONIE

(30) Priorität: 10.08.2001 DE 10138275
(43) Veröffentlichungstag der Anmeldung: 19.05.2004
(73) Patentinhaber: Boehringer Ingelheim Pharma GmbH & Co.KG, 55216 Ingelheim am Rhein (DE)
(72) Erfinder: LEHR, Erich, 55425 Waldalgesheim (DE); MIERAU, Joachim, 55127 Mainz (DE); PIEPER, Michael, Paul, 55411 Bingen (DE)
(86) Internationale Anmeldenummer: PCT/EP2002/008691
(87) Internationale Veröffentlichungsnummer: WO 2003/013521

(56) Entgegenhaltungen:
- WO-A-00/61156
- WO-A-98/51310
- US-A- 5 177 081
- WILLNER P; LAPPAS, S; CHEETA, S; MUSCAT, R: "Reversal of stress-induced anhedonia by the dopamine receptor agonists, pramipexole" PSYCHOPHARMACOLOGY, Bd. 115, 1994, Seiten 454-462, XP009002717
- LYNCH, M R: "Selective effects on prefrontal cortex serotonin by dopamine D3 receptor agonism: interaction with low-dose haloperidol" PROG. NEURO-PSYCHOPHARMACOL. & BIOL. PSYCHIAT., Bd. 21, 1997, Seiten 1141-1153, XP001120814

## Beschreibung

Die Erfindung betrifft die Verwendung von Dopaminagonisten zur Herstellung eines Arzneimittels zur Beseitigung und/oder Linderung der Anhedonie, beim Entzug von Abhängigkeitserkrankungen

### Hintergrund der Erfindung

Der Begriff der Anhedonie wird im Stand der Technik zur Bezeichnung einer Reihe symptomatischer Zustände verwendet. So bezeichnet Anhedonie beispielsweise den Verlust der Lebensfreude sowie die Unfähigkeit, Freude durch Erlebnisse oder Anregungen zu empfinden, die normalerweise Vergnügen bereiten. Anhedonie wird gelegentlich in soziale Anhedonie (beispielsweise den Verlust der Freude, mit Freunden zusammen zu sein) und psychische Anhedonie (beispielsweise den Verlust der Freude an der Beobachtung der Schönheit der Natur) eingeteilt. Als Symptom findet sich Anhedonie bei psychiatrischen Krankheitsbildern wie der majoren Depression, der Schizophrenie sowie bei Abhängigkeitserkrankungen. Gegebenenfalls tritt Anhedonie auch als Folge schwerer Belastungen und Extremsituationen auf.

### Beschreibung der Erfindung

Es wurde nun überraschenderweise gefunden, daß Dopaminagonisten zur Beseitigung und/oder Linderung der Anhedonie beim Entzug von Abhängigkeitserkrankungen in therapeutisch wirksamen Dosen sinnvoll zum Einsatz gelangen können.

Dementsprechend zielt die vorliegende Erfindung auf die Verwendung von Dopaminagonisten zur Herstellung eines Arzneimittels zur Beseitigung und/oder Linderung der Anhedonie beim Entzug von Abhängigkeitserkrankungen.

Unter Abhängigkeitserkrankungen werden im Rahmen der vorliegenden Erfindung Erkrankungen oder Störungen des Gesundheitszustandes verstanden, die sich aus der physischen und/oder psychischen Abhängigkeit eines Individuums von beispielsweise Medikamenten und/oder Drogen ergeben.
Medikamentenabhängigkeit kann sich beispielsweise ergeben durch regelmäßige Einnahme von Wirkstoffen, wie beispielsweise Opiaten. Drogenabhängigkeit kann sich beispielsweise ergeben durch regelmäßige Einnahme von Heroin, Cocain, Marihuana und ähnlichem. Unter Drogenabhängigkeit wird im Rahmen der vorliegenden Erfindung ferner verstanden, die physische und/oder psychische Abhängigkeit von Alkohol, Coffein oder Nikotin durch regelmäßigen Konsum alkoholischer oder Coffein-haltiger Getränke und Tabakwaren.
Unter Abhängigkeit im Sinne der vorliegenden Erfindung werden auch generelle, nicht substanzbedingte Abhängigkeiten verstanden, wie sie beispielsweise bei Eßsucht oder Spielsucht beobachtbar sind.

Der Entzug von gewohnten, belohnenden Auslösern führt in der Regel zu einer Reihe von pathologischen psychophysiologischen Reaktionen. Im Stand der Technik sind Therapieansätze bekannt, bei denen versucht wird, eine Substitution der ursprünglichen Suchtauslöser durch andere, weniger schädigende Substanzen durchzuführen. Diese sollen den Entzug mildern, ohne selbst die Abhängigkeit zu fördern. Als zielgerichteter wird angesehen, die Symptomatik der Abhängigkeit genauer zu analysieren und diese dann zielgerichtet zu beheben. Dies bedeutet zwar zunächst nur eine symptomatische Behandlung, durch die Befreiung vom Zwang der immer wieder neuen Einnahme der suchterzeugenden Agentien erhält der Organismus aber die benötigte Zeit zur längerfristigen Selbstheilung.

Beim Entzug treten vor allem Erregungs- und Unruhezustände sowie ausgeprägte Anhedonie auf. Während bei ersteren mit entsprechenden präklinischen Ansätzen bereits Therapieansätze versucht wurden, konnte bisher mangels geeigneter präklinischer Modelle die Anhedonie nicht gezielt therapiert werden.
Hier greift die vorliegende Erfindung: In einem neu entwickelten Versuch kann präklinisch erstmals Anhedonie bei Tieren wahrscheinlich gemacht werden, und überraschenderweise haben gerade in diesem Modell die beanspruchten Substanzen überzeugend gewirkt. Sie behoben die Anhedonie-Symptome mit überzeugender Reproduzierbarkeit in ungewöhnlich niedrigem Dosisbereich. Bisher konnte eine derartig überzeugenden Wirkung bei keiner anderen Substanz nachgewiesen werden.

Bevorzugte im Rahmen der vorliegenden Erfindung einsetzbare Dopaminagonisten sind ausgewählt aus der Gruppe bestehend aus Pramipexol, Talipexol, Ropinirol, Apomorphin, Lisurid, Tergurid, Pergolid, Cabergolin, Bromocriptin, Ropinirol, (-)Quinpirol und (+)-7-OH-DPAT gegebenenfalls in Form ihrer Enantiomere, gegebenfalls in Form der pharmakologisch verträglichen Säureadditionssalze sowie gegebenenfalls in Form der Hydrate und Solvate.

Besonders bevorzugte Dopaminagonisten im Rahmen der erfindungsgemäßen Anwendung sind ausgewählt aus der Gruppe bestehend aus Pramipexol, Talipexol, und Ropinirol, gegebenenfalls in Form ihrer Enantiomere, gegebenfalls in Form der pharmakologisch verträglichen Säureadditionssalze sowie gegebenenfalls in Form der Hydrate und Solvate.

Von herausragender Bedeutung im Rahmen der erfindungsgemäßen Anwendung sind die Dopaminagonisten ausgewählt aus Pramipexol und Talipexol, gegebenenfalls in Form ihrer Enantiomere, gegebenfalls in Form der pharmakologisch verträgliche Säureadditionssalze sowie gegebenenfalls in Form der Hydrate und Solvate.

Eine Bezugnahme auf einen der vorstehend genannten Dopaminagonisten schließt eine Bezugnahme auf die gegebenenfalls existierenden Enantiomere der jeweiligen Verbindung mit ein. Beispielsweise schließt eine Bezugnahme auf Pramipexol die Bezugnahme auf das (+)-Enantiomer sowie auf das (-)-Enantiomer mit ein. Im Rahmen der vorliegenden Erfindung kommt allerdings dem (-)-Enantiomer eine besondere Bedeutung zu.

Die erfindungsgemäß einsetzbaren Dopaminagonisten können gegebenenfalls in Form ihrer pharmazeutisch verträglichen Säureadditionssalze sowie gegebenenfalls in Form ihrer Hydrate und/oder Solvate verwendet werden. Unter pharmazeutisch verträglichen Säureadditionssalzen der Dopaminagonisten werden erfindungsgemäß solche Salze verstanden, die ausgewählt sind aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure, wobei die Salze der Salzsäure, Bromwasserstoffsäure, Schwefelsäure, Phosphorsäure, und Essigsäure besonders bevorzugt sind. Eine besondere Bedeutung kommt hierbei den Salzen der Salzsäure zu.

Im Falle des erfindungsgemäß besonders bevorzugt einsetzbaren Pramipexols gelangen ebenfalls bevorzugt die Hydrochloride zur Anwendung, wobei diesbezüglich dem Pramipexoldihydrochlorid eine besondere Bedeutung zukommt. Von den Hydraten des Pramipexols ist das Pramipexoldihydrochlorid-monohydrat besonders bevorzugt.

Die erfindungsgemäß einsetzbaren Dopaminagonisten können gegebenenfalls in Kombination mit weiteren Wirkstoffen zur Anwendung gelangen. Bevorzugte Kombinationspartner sind Verbindungen ausgewählt aus den Klassen der Antidepressiva, Anxiolytica und Sedativa. Über synergistische Effekte bei der beabsichtigten Wirkung können im Falle des Einsatzes von Kombinationen enthaltend neben Dopaminagonisten einen der vorstehend genannten weiteren Wirkstoffe die Dosierung der Einzelkomponenten verringert werden.

Die Dosierung der Dopaminagonisten ist naturgemäß stark abhängig von der Stärke der zu therapierenden Symptomatik einerseits sowie von der Wahl des Wirkstoffs andererseits. Beispielsweise und ohne den Gegenstand der vorliegenden Erfindung darauf zu beschränken seien an dieser Stelle mögliche Dosierungen insbesondere für die erfindungsgemäß besonders bevorzugte Verbindung Pramipexol angegeben. Pro Tag kann diese in Dosierungen von etwa 0,05 bis 3 mg, bevorzugt von etwa 0,1 bis 1,5 mg Verwendung finden. Diese Dosierungen sind bezogen auf Pramipexol in Form seiner freien Base. Bezogen auf die bevorzugt zum Einsatz gelangende Salzform Pramipexoldihydrochlorid-monohydrat entsprechen die vorstehend genannten Dosierungen etwa 0,07 bis 4,26 mg, bevorzugt 0,14 bis 2,13 mg Pramipexoldihydrochlorid-monohydrat pro Tag.

Eine mögliche und nur als beispielhaft erläuternd zu verstehende Vorgehensweise zur Dosierung ist nachfolgend ausgeführt (bezogen auf Pramipexol in Form seiner freien Base): Individuelle Dosistitration in wöchentlichen Abständen je nach Wirkung und Verträglichkeit.
1. Woche: 3mal täglich 1 Tablette enthaltend 0,088 mg Pramipexol;
2. Woche: 3mal täglich 1 Tablette enthaltend 0,18 mg Pramipexol;
3. Woche und folgende: 3mal täglich 1/2 Tablette enthaltend 0,7 mg Pramipexol.

Die Dopaminagonisten können im Rahmen der erfindungsgemäßen Anwendung oral, transdermal, intrathecal, inhalativ, nasal oder parenteral verabreicht werden. Geeignete Anwendungsformen sind beispielsweise Tabletten, Kapseln, Zäpfchen, Lösungen, Säfte, Emulsionen, dispersible Pulver oder Pflaster. Bezüglich möglicher Ausführungsformen einer erfindungsgemäß einsetzbaren transdermalen Applikationsform wird an dieser Stelle insbesondere bezüglich Pramipexol auf die Ausführungsbeispiele gemäß US 5112842 verwiesen, auf die hiermit ausdrücklich Bezug genommen wird. Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Im Folgenden sind einige Beispiele für erfindungsgemäß einsetzbare pharmazeutische Zubereitungen angegeben. Diese dienen lediglich der beispielhaften Erläuterung.

**Tablette 1:**

| Bestandteile: | mg |
|---|---|
| Pramipexoldihydrochlorid-monohydrat | 1,00 |
| Mannitol | 121,50 |
| Maisstärke | 79,85 |
| Hochdisperses Siliciumdioxid, wasserfrei | 2,30 |
| Polyvidon K25 | 2,35 |
| Magnesiumstearat | 3,00 |
| Gesamt | 210,00 |

**Tablette 2:**

| Bestandteile: | mg |
|---|---|
| Pramipexol | 0,5 |
| Mannitol | 122,0 |
| Maisstärke, getrocknet | 61,8 |
| Maisstärke | 18,0 |
| Hochdisperses Siliciumdioxid, wasserfrei | 2,4 |
| Polyvidon K25 | 2,3 |
| Magnesiumstearat | 3,0 |
| Gesamt | 210,0 |

**Tablette 3:**

| Bestandteile: | mg |
|---|---|
| Pramipexol | 0,25 |
| Mannitol | 61,00 |
| Maisstärke | 39,90 |
| Hochdisperses Siliciumdioxid, wasserfrei | 1,20 |
| Polyvidon K25 | 1,15 |
| Magnesiumstearat | 1,5 |
| Gesamt | 105,00 |

**Tablette 4:**

| Bestandteile: | mg |
|---|---|
| Pramipexol | 0,125 |
| Mannitol | 49,455 |
| Maisstärke getrocknet | 25,010 |
| Maisstärke | 7,300 |
| Hochdisperses Siliciumdioxid, wasserfrei | 0,940 |
| Polyvidon K25 | 0,940 |
| Magnesiumstearat | 1,230 |
| Gesamt | 85,000 |

### Lösung zur Injektion:

| | |
|---|---|
| Pramipexoldihydrochlorid-monohydrat | 0,3 mg |
| Natriumchlorid | 0,8 mg |
| Benzalkoniumchlorid Aqua ad injectionem ad 100 ml | 0,01 mg |

## Patentansprüche

1. Verwendung von Dopaminagonisten zur Herstellung eines Arzneimittels zur Beseitigung und/oder Linderung der Anhedonie beim Entzug von Abhängigkeitserkrankungen.

2. Verwendung nach Anspruch 1, **dadurch gekennzeichnet, dass** die Abhängigkeitserkrankung ausgewählt ist aus Medikamentenabhängigkeit, Drogenabhängigkeit, Alkoholabhängigkeit, Koffeinabhängigkeit oder Nikotinabhängigkeit.

3. Verwendung nach Anspruch 1, **dadurch gekennzeichnet, dass** die Abhängigkeitserkrankung ausgewählt ist aus Eßsucht ist oder Spielsucht.

4. Verwendung nach einem der Ansprüche 1, 2 oder 3, **dadurch gekennzeichnet, daß** ein oder mehrere, bevorzugt ein Dopaminagonist ausgewählt aus der Gruppe bestehend aus Pramipexol, Talipexol, Ropinirol, Apomorphin, Lisurid, Tergurid, Pergolid, Cabergolin, Bromocriptin, (-)Quinpirol und (+)-7-OH-DPAT gegebenenfalls in Form ihrer Enantiomere, gegebenfalls in Form der pharmakologisch verträglichen Säureadditionssalze sowie gegebenenfalls in Form der Hydrate und Solvate verwendet werden.

5. Verwendung nach Anspruch 3, wobei der Dopaminagonist ausgewählt ist aus der Gruppe bestehend aus Pramipexol, Talipexol, und Ropinirol, gegebenenfalls in Form ihrer Enantiomere, gegebenenfalls in Form der pharmakologisch verträglichen Säureadditionssalze sowie gegebenenfalls in Form der Hydrate und Solvate.

6. Verwendung nach Anspruch einem der Ansprüche 1 bis 5, wobei der Dopaminagonist Pramipexol ist, gegebenenfalls in Form seines (+) oder (-) Enantiomeren, gegebenenfalls in Form der pharmakologisch verträglichen Säureadditionssalze sowie gegebenenfalls in Form der Hydrate und Solvate.

## Claims

1. Use of dopamine agonists for preparing a pharmaceutical composition for overcoming and/or alleviating anhedonia during withdrawal from dependency diseases.

2. Use according to claim 1, **characterised in that** the dependency disease is selected from among prescription drug dependency, drug dependency, alcohol dependency, caffeine dependency or nicotine dependency.

3. Use according to claim 1, **characterised in that** the dependency disease is selected from among compulsive eating or compulsive gambling.

4. Use according to one of claims 1, 2 or 3, **characterised in that** one or more, preferably one dopamine agonist selected from among pramipexole, talipexole, ropinirol, apomorphine, lisuride, terguride, pergolide, cabergoline, bromocriptine, (-)quinpirol and (+)-7-OH-DPAT, is or are used, optionally in the form of their enantiomers, optionally in the form of the pharmacologically acceptable acid addition salts thereof and optionally in the form of the hydrates and solvates thereof.

5. Use according to claim 3, wherein the dopamine agonist is selected from among pramipexole, talipexole and ropinirol, optionally in the form of their enantiomers, optionally in the form of the pharmacologically acceptable acid addition salts thereof and optionally in the form of the hydrates and solvates thereof.

6. Use according to claim one of claims 1 to 5, wherein the dopamine agonist is pramipexole, optionally in the form of its (+) or (-) enantiomers, optionally in the form of the pharmacologically acceptable acid addition salts thereof and optionally in the form of the hydrates and solvates thereof.

## Revendications

1. Utilisation d'agonistes de la dopamine pour la préparation d'un médicament destiné à supprimer et/ou à soulager l'anhédonie lors de cures de désintoxication dans le cadre de maladies de dépendance.

2. Utilisation selon la revendication 1, **caractérisée en ce que** la maladie de dépendance est choisie parmi la dépendance aux médicaments, la dépendance aux drogues, la dépendance à l'alcool, la dépendance à la caféine ou à la nicotine.

3. Utilisation selon la revendication 1, **caractérisée en ce que** la maladie de dépendance est choisie parmi la boulimie et l'addiction au jeu.

4. Utilisation selon l'une des revendications 1, 2 ou 3, **caractérisée en ce qu'**on utilise un ou plusieurs, de préférence un agoniste de la dopamine, choisi dans le groupe formé par le Pramipexol, le Talipexol, le Ropinirol, l'Apomorphine, le Lisuride, le Terguride, le Pergolide, la Cabergoline, la Bromocriptine, le(-)Quinpirol et le (+)-7-OH-DPAT, éventuellement sous la forme de leurs énantiomères, éventuellement sous la forme de sels d'addition d'acide pharmacologiquement compatibles, ainsi qu'éventuellement sous forme d'hydrates ou de solvates.

5. Utilisation selon la revendication 3, dans laquelle l'agoniste de la dopamine est choisi dans le groupe formé par le Pramipexol, le Talipexol et le Ropinirol, éventuellement sous forme de leurs énantiomères, éventuellement sous forme des sels d'addition d'acide pharmacologiquement compatibles, ainsi qu'éventuellement sous forme d'hydrates ou de solvates.

6. Utilisation selon l'une des revendications 1 à 5, dans laquelle l'agoniste de la dopamine est le Pramipexol, éventuellement sous la forme de ses énantiomères (+) ou (-), éventuellement sous la forme des sels d'addition d'acide pharmacologiquement compatibles, ainsi qu'éventuellement sous la forme d'hydrates ou de solvates.
